(19) European Patent Office / Europäisches Patentamt / Office européen des brevets

(11) **EP 2 702 948 B1**

(12) **EUROPEAN PATENT SPECIFICATION**

(45) Date of publication and mention
of the grant of the patent:
**01.05.2019  Bulletin 2019/18**

(51) Int Cl.:
**A61B 8/00** *(2006.01)*          **A61B 8/08** *(2006.01)*

(21) Application number: **13181765.2**

(22) Date of filing: **27.08.2013**

(54) **Object information acquisition apparatus, display method**

Objektinformationserfassungsvorrichtung, Anzeigeverfahren

Appareil d'acquisition d'informations d'objet et procédé d'affichage

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.08.2012  JP 2012187616**

(43) Date of publication of application:
**05.03.2014  Bulletin 2014/10**

(73) Proprietor: **Canon Kabushiki Kaisha
Tokyo 146-8501 (JP)**

(72) Inventor: **Nagae, Kenichi
Tokyo 146-8501 (JP)**

(74) Representative: **TBK
Bavariaring 4-6
80336 München (DE)**

(56) References cited:
**EP-A1- 2 702 945          EP-A1- 2 702 946
EP-A2- 1 097 674          JP-A- H07 397
JP-A- H10 127 628          JP-A- 2012 061 206
US-A- 5 471 989          US-A1- 2011 307 181
US-A1- 2012 022 373**

- **F. VIGNON ET AL: "Capon beamforming in
medical ultrasound imaging with focused
beams", IEEE TRANSACTIONS ON
ULTRASONICS, FERROELECTRICS AND
FREQUENCY CONTROL, vol. 55, no. 3, 1 March
2008 (2008-03-01), pages 619-628, XP055091879,
ISSN: 0885-3010, DOI: 10.1109/TUFFC.2008.686**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Field of the Invention

The present disclosure relates to an object information acquisition apparatus, a display method. In particular, the present disclosure relates to a technique for transmitting elastic waves to an object, and displaying distribution information acquired by receiving reflected waves from the object.

Description of the Related Art

In the field of an ultrasonograph which is an object information acquisition apparatus, the ultrasonograph is known to transmit ultrasonic waves (elastic waves) to an object, receives reflected waves reflected inside the object, and acquires an ultrasonic echo image, based on the pulse echo method. Japanese Patent Application Laid-Open No. 2012-24133 discusses an apparatus for generating an ultrasonic image (especially moving image) by applying delay and sum processing, envelope detection, etc. to a plurality of reception signals acquired by receiving ultrasonic waves. With the apparatus discussed in Japanese Patent Application Laid-Open No. 2012-24133, when a user specifies an area to be enlarged as a Region Of Interest (ROI), an enlarged image of the specified area is displayed on a display unit. The user can specify whether filtering is applied to data of the enlarged image.

With the apparatus discussed in Japanese Patent Application Laid-Open No. 2012-24133, the displayed enlarged image is acquired by applying envelope detection to scanning line signals (echo data) that have undergone delay and sum processing, as with the image before enlargement. However, an image acquired through such processing is considered to provide limited visibility even after enlargement.

Prior art which is related to this field of technology can be found e.g. in document US 5,471,989 A disclosing ultrasonic diagnostic imaging with enhanced zoom, document US 2012/0022373 A1 disclosing a measurement apparatus which performs imaging using adaptive signal processing, and spatial smoothing, document US 2011/0307187 A1 disclosing an ultrasonic apparatus with an adaptive signal processing block and a fixed signal processing block, and in document JP H07 397 A disclosing an ultrasonic diagnostic system which concurrently displays the dynamic image of the original image and the dynamic image of the zoom image.

SUMMARY OF THE INVENTION

An embodiment of the present invention is directed to a technique for displaying on a display unit an enlarged image having higher resolution when displaying the enlarged image.

According to aspects of the present invention, there are provided an object information acquisition apparatus and a display method as specified in the respective claims.

Further features and aspects of the present invention will become apparent from the following detailed description of exemplary embodiments with reference to the attached drawings.

BRIEF DESCRIPTION OF THE DRAWINGS

Fig. 1 is a block diagram illustrating an overview of an object information acquisition apparatus according to an exemplary embodiment of the present invention.

Fig. 2 is block diagrams illustrating a configuration of a fixed signal processing block.

Figs. 3A, 3B, and 3C are block diagrams illustrating different configurations of an adaptive signal processing block.

Fig. 4 is a flowchart illustrating processing of a display method according to a first exemplary embodiment.

Fig. 5 illustrates an example screen displayed on a display unit according to the first exemplary embodiment.

Fig. 6 illustrates example images displayed on the display unit according to the first exemplary embodiment, and an image displayed after envelope detection for comparison.

Fig. 7 illustrates an example screen displayed on the display unit according to a second exemplary embodiment.

Fig. 8 illustrates an example screen displayed on the display unit according to the second exemplary embodiment.

Fig. 9 is a graph illustrating a relation between an enlargement rate and a combination rate according to a third exemplary embodiment.

DESCRIPTION OF THE EMBODIMENTS

Exemplary embodiments of the present invention will be described below with reference to the accompanying drawings. Basically, identical elements are assigned the same reference numerals, and redundant descriptions will be omitted.

In the present exemplary embodiment, an elastic wave typically refers to an ultrasonic wave and includes what is called sound wave, ultrasonic wave, or acoustic wave. The object information acquisition apparatus according to the present exemplary embodiment includes an apparatus that transmits elastic waves to an object, receives reflected waves (reflected elastic waves) reflected inside the object, and acquires intra-object distribution information as image data. The acquired intra-object distribution information is information reflecting the acoustic impedance difference between intra-object tissues. In the present exemplary embodiment, the scanning line indicates a virtual line formed in the traveling direction of elastic waves transmitted from a probe.

A basic apparatus configuration and a processing flow according to the present exemplary embodiment will be described below.

<Basic configuration of object information acquisition apparatus>

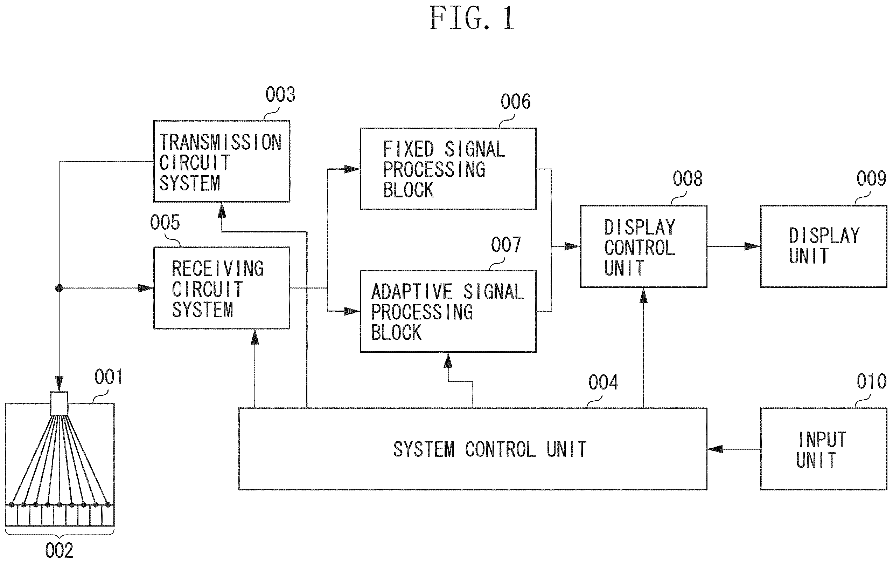

A configuration of an object information acquisition apparatus according to the present exemplary embodiment of the present invention will be described below with reference to Fig. 1. Fig. 1 is a block diagram illustrating an overview of the object information acquisition apparatus according to the present exemplary embodiment. The object information acquisition apparatus according to the present exemplary embodiment includes a probe 001 having a plurality of conversion elements 002, a receiving circuit system 005, a transmission circuit system 003, a fixed signal processing block 006, an adaptive signal processing block 007, and a display control unit 008. The object information acquisition apparatus according to the present exemplary embodiment further includes a display unit 009, an input unit 010, and a system control unit 004.

The probe 001 is a receiver transmitter for transmitting elastic waves to a plurality of intra-object positions, and receives reflected waves. The probe 001 includes the plurality of conversion elements 002 for converting elastic waves into electrical signals.

The transmission circuit system 003 is a transmission signal generation unit for generating, based on a control signal from the system control unit 004, a plurality of transmission signals having a delay time and an amplitude for each target position and each target direction. The plurality of conversion elements 002 converts the transmission signals into elastic waves, and the probe 001 transmits the elastic waves to the object as elastic wave beams. The plurality of conversion elements 002 also receives elastic waves (reflected waves) reflected by intra-object subjects (reflective interfaces and reflectors), and converts the elastic waves into a plurality of receiving signals. The receiving signals are input to the receiving circuit system 005.

The receiving circuit system 005 is a receiving signal processing unit for amplifying the plurality of receiving signals and converting the receiving signals into a plurality of digital signals (digitized receiving signals). In the present exemplary embodiment, not only analog receiving signals output by the conversion elements 002 but also amplified and digitally converted signals are referred to as receiving signals. The plurality of digital signals output from the receiving circuit system 005 is input to the fixed signal processing block 006 and the adaptive signal processing block 007.

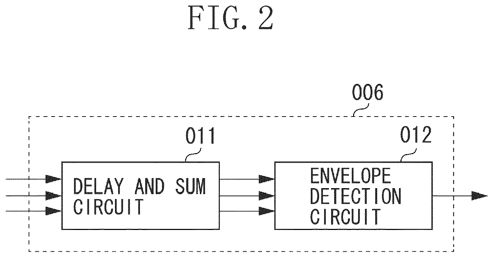

The fixed signal processing block 006 is equivalent to a fixed signal processing unit according to the present exemplary embodiment. Fig. 2 illustrates a configuration of the fixed signal processing block 006. In the fixed signal processing block 006, a delay and sum circuit 011 (i.e., delay and sum unit) applies delay processing to the plurality of digital signals according to transmission directions and positions of the elastic waves, and then applies addition to the plurality of digital signals. In other words, a delay and sum processing is performed. A plurality of scanning line signals is acquired by delay and sum processing. The fixed signal processing block 006 may multiply each of the plurality of digital signals by a weight before applying delay and sum processing to the digital signals. Although the weight changes according to observation positions and transmission and reception conditions, a predetermined (fixed) weight is used in many cases. Delay and sum processing generates signals corresponding to the sound pressure of reflected waves reflected at respective intra-object positions, as scanning line signals. Then, the envelope detection circuit 012 (envelope detection unit) applies envelope detection to the plurality of scanning line signals to acquire first distribution information. The fixed signal processing block 006 outputs the acquired first distribution information to the display control unit 008.

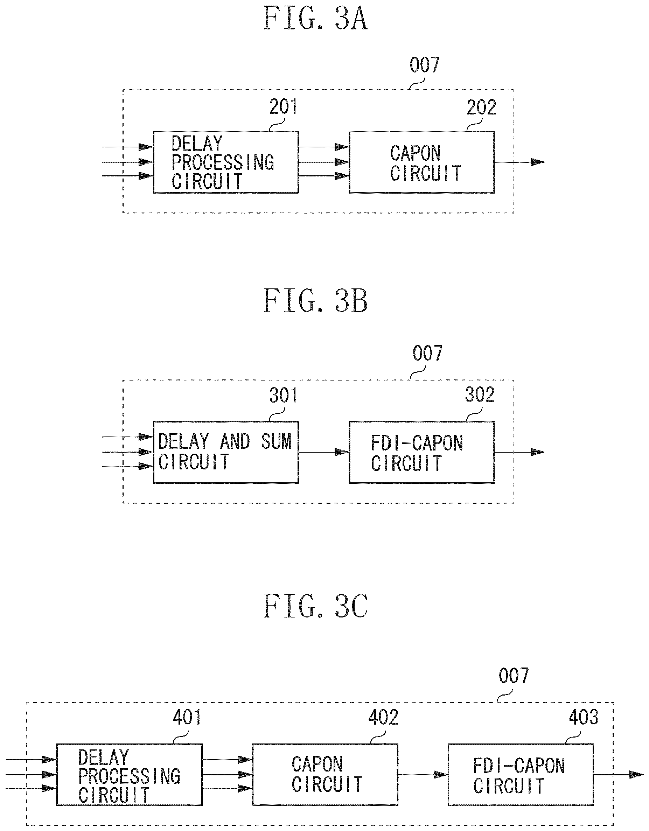

The adaptive signal processing block 007 is equivalent to an adaptive signal processing unit according to an embodiment of the present invention. Adaptive signal processing adaptively changes relevant processing parameters according to the receiving signals. In particular, the Capon method (also referred to as Constrained Minimization of Power (CMP)), one of adaptive signal processing methods, is applied to a plurality of input signals so that the electric power is minimized with fixed sensitivity for the target directions and target positions. Such adaptive signal processing has an effect of improving the spatial resolution. The adaptive signal processing block 007 outputs as second distribution information the power strength distribution having an improved resolution in at least one of the depth direction and the direction perpendicular to the depth direction. The depth direction refers to the traveling direction of the elastic waves (ultrasonic beams) transmitted from the probe 001, and equals to the scanning line direction. Adaptive signal processing will be described in detail below with reference to Figs. 3A, 3B, and 3C.

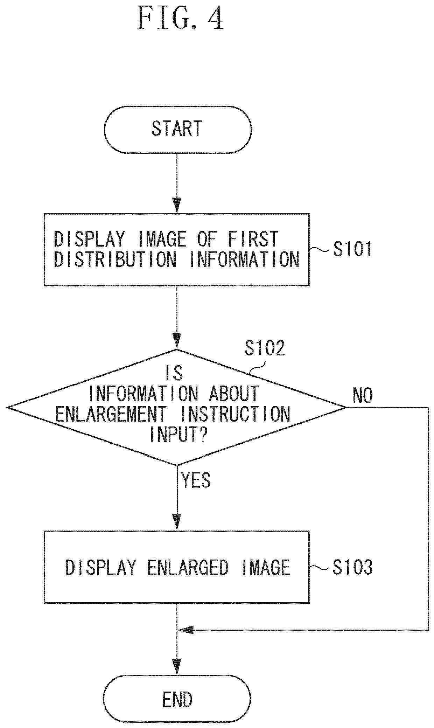

The display control unit 008 inputs the first distribution information from the fixed signal processing block 006, and the second distribution information from the adaptive signal processing block 007. The display control unit 008 outputs image information for displaying distribution information on the display unit 009. The display unit 009 displays an image indicating intra-object distribution information based on the image information output from the display control unit 008. The processing performed by the display control unit 008 will be described in detail below with reference to Fig. 4. The display control unit 008 applies various kinds of image processing, such as edge emphasis and contrast adjustment to image information of the first distribution information, image information of the second distribution information, and image information for a combination of the first and second distribution information, and outputs image information of luminance data.

In the present exemplary embodiment, the fixed signal processing block 006, the adaptive signal processing block 007, the display control unit 008, and the system control unit 004 are configured of processing devices such as a central processing unit (CPU), a graphics processing unit (GPU), and a field programmable gate array (FPGA) chip. The display unit 009 displays an image based on the image information input from the display control unit 008. The display unit 009 is a liquid crystal display (LCD), a cathode ray tube (CRT), or an organic electroluminescence (EL) display.

The input unit 010 is used by a user to input an enlargement instruction. The user input an enlargement instruction by using the input unit 010, referring to an image of the first distribution information displayed on the display unit 009. The input unit 010 is a pointing device, such as a mouse and a keyboard, a pen tablet, or a touchpad attached to the surface of the display unit 009. The input unit 010 may also be a dial or button for specifying an enlargement rate provided on the apparatus. The display unit 009 and the input unit 010 may be connected to the object information acquisition apparatus according to the present exemplary embodiment, instead of being included in the object information acquisition apparatus according to the present exemplary embodiment.

<Details of adaptive signal processing>

Processing performed by the adaptive signal processing block 007 of the present exemplary embodiment will be described below. Figs. 3A, 3B, and 3C illustrate three different configurations of the adaptive signal processing block 007. Example configurations of the adaptive signal processing block 007 according to the present exemplary embodiment will be described below with reference to Figs. 3A, 3B, and 3C.

Fig. 3A illustrates a configuration of the adaptive signal processing block 007 for improving the resolution in the direction perpendicular to the depth direction (traveling direction of the elastic waves (ultrasonic beams) transmitted from the probe 001). M. SASSO et al., Medical Ultrasound Imaging Using The Fully Adaptive Beamformer, Proc. Acoustics, Speech Signal Process. volume. 2, pp. 489-492 (Mar. 2005) discusses a technique of such adaptive signal processing for improving the resolution in the direction perpendicular to the depth direction.

Processing performed when adaptive signal processing is applied to the plurality of receiving signals will be described below based on the Capon method as an example.

Processing for calculating a correlation matrix based on the plurality of receiving signals will be described below. First of all, the delay processing circuit 201 applies the Hilbert transform and the delay processing (phasing processing) according to target positions to the plurality of receiving signals output from the plurality of conversion elements 002. The receiving signals in the complex representation are calculated in this way. When the s-th sample of a signal obtained by processing a receiving signal from the k-th element is xk[s], an input vector X[s] of the s-th sample is defined by the following formula.

$$X[s] = [x_1[s], x_2[s], \cdots, x_M[s]]^T \qquad \cdots \cdots \quad (1)$$

where M indicates the number of elements.

Then, a Capon circuit 202 (adaptive signal processing unit) calculates a correlation matrix $R_{xx}$ by using the input vector X[s].

$$R_{xx} = E\left[X[s]X^H[s]\right]$$

$$= \begin{bmatrix} E\left[x_1[s]x_1^*[s]\right] & E\left[x_1[s]x_2^*[s]\right] & \cdots & E\left[x_1[s]x_M^*[s]\right] \\ E\left[x_2[s]x_1^*[s]\right] & E\left[x_2[s]x_2^*[s]\right] & \cdots & E\left[x_2[s]x_M^*[s]\right] \\ \vdots & \vdots & \ddots & \vdots \\ E\left[x_M[s]x_1^*[s]\right] & E\left[x_M[s]x_2^*[s]\right] & \cdots & E\left[x_M[s]x_M^*[s]\right] \end{bmatrix} \quad \cdots \cdots \quad (2)$$

where the superscript H indicates a complex conjugate transposition, and the superscript * indicates a complex conjugate. $E[\cdot]$ indicates processing for calculating a time average, i.e., processing for varying the sample number (s in this case) and calculating an average.

Then, to suppress the effect of a correlated interference wave which reaches the probe 001 from other than target directions, the Capon circuit 202 applies the spatial averaging method to the correlation matrix $R_{xx}$ to obtain an average correlation matrix $R'_{xx}$.

$$R'_{xx} = \sum_{n=1}^{M-K+1} z_n R_{xx}^n \quad \cdots \cdots \quad (3)$$

where $R_{xx}^n$ indicates a partial matrix in the correlation matrix $R_{xx}$, moving along the diagonal elements of $R_{xx}$. Specifically, $R_{xx}^n$ is a matrix having a size of K x K, positioned so that the (n, n) element of $R_{xx}$ equals to the first diagonal element of $R_{xx}^n$. $Z_n$ indicates a coefficient used when adding respective partial matrices, and is adjusted so that the sum total of $Z_n$ equals 1.

The Capon method obtains a complex weight for minimizing the output power under certain restriction conditions. The complex weight refers to a weight represented by a complex vector. With the Capon method, an optimum complex weight $W_{opt}$ for minimizing the output power, with the sensitivity for the receiving signals of the elastic waves from the target directions restrained to 1, can be calculated by the following formula.

$$W_{opt} = \gamma R'^{-1}_{xx}C, \qquad \gamma = \frac{1}{C^H R'^{-1}_{xx}C} \quad \cdots \cdots \quad (4)$$

where C indicates a restriction vector which varies according to the element position and target direction. However, when the phasing delay processing has been applied to the receiving signals, C may be a vector having all values of 1 with respect to the size (K in this case) of the average correction matrix.

A calculated electric power $P_{min}$ can be obtained as follows by using the complex weight $W_{opt}$. The calculated electric power $P_{min}$ indicates distribution information (information about distribution related to the acoustic characteristics) reflecting the acoustic impedance difference between intra-object tissues according to the present exemplary embodiment.

$$P_{min} = \frac{1}{2}\frac{1}{C^H R'^{-1}_{xx}C} \quad \cdots \cdots \quad (5)$$

The Capon circuit 202 can acquire a correlation matrix and further an average correction matrix based on the receiving signals, and, by using an inverse matrix, acquire a complex weight and a power distribution by using the complex weight. The complex weight and the electric power by using the complex weight are a complex weight and an electric power when the sensitivity is set to 1 for signals of the elastic waves from the target directions, and signals of the elastic waves reaching from other directions are suppressed. In other words, the Capon method enables selectively extracting signals of the elastic waves from the target directions, resulting in an improved spatial resolution in the direction perpendicular to the depth direction.

The electric power can also be calculated by applying QR decomposition and backward substitution to the average correction matrix, without directly obtaining an inverse matrix. The adaptive signal processing block 007 applies to the plurality of receiving signals in this way adaptive signal processing (using the Capon method) with a weight adaptively changing according to the receiving signals. As a result, the adaptive signal processing block 007 outputs a signal strength distribution (equivalent to the second distribution information) having an improved spatial resolution in the direction perpendicular to the depth direction.

A second example configuration of the adaptive signal processing block 007 will be described below with reference to Fig. 3B.

Fig. 3B illustrates a configuration of the adaptive signal processing block 007 for improving the resolution in the depth direction, i.e., the traveling direction of the elastic waves (ultrasonic beams) transmitted from the probe 001. As a technique for improving the spatial resolution in the depth direction, adaptive signal processing is combined with the Frequency Domain Interferometry (FDI) method. Hirofumi Taki, Kousuke Taki, Takuya Sakamoto, Makoto Yamakawa, Tsuyoshi Shiina and Toru Sato: Conf Proc IEEE Eng Med Biol Soc. 2010; 1: 5298-5301 discusses a technique in which the FDI method and the Capon method (adaptive signal processing) are applied.

The FDI method decomposes the receiving signals into frequency components, and varies the phase of the decomposed signals according to the target positions to presume the received electric power at the target positions. Phase variation can be predetermined based on the product of the distance from a certain reference position to the target positions and the number of waves corresponding to the frequency.

In other words, a method combining the FDI method and adaptive signal processing will presume the received electric power at the target positions by using phase variation and weight calculated for each signal through adaptive signal processing, instead of predetermined fixed phase variation and weight, with respect to each receiving signal decomposed into frequency components.

When applying the frequency averaging technique to the receiving signals of the elastic waves having a wide frequency band as with pulse waves, whitening is preferably applied to the receiving signals based on a reference signal.

Referring to Fig. 3B, the delay and sum circuit 301 (delay and sum unit) applies the delay processing to the plurality of digital signals according to the transmission directions and positions of the elastic waves, and applies delay and sum processing to the plurality of digital signals after the delay processing. Similar to the delay and sum processing in the fixed signal processing block 006, the delay and sum processing in the adaptive signal processing block 007 generates signals corresponding to the sound pressure of the reflected waves reflected at respective intra-object positions, as scanning line signals.

Then, an FDI-Capon circuit 302 (FDI adaptive processing unit) receives as input signals the scanning line signals output from the delay and sum circuit 301. Then, the FDI-Capon circuit 302 extracts signals for the time interval of one unit of processing, i.e., the processing range, based on the plurality of scanning line signals.

Then, the FDI-Capon circuit 302 applies the Fourier transform to the extracted signals to decompose the signals into frequency components ($X_{s1}$, $X_{s2}$, $X_{s3}$, ..., and $X_{sN}$). In the meantime, the FDI-Capon circuit 302 inputs at least one reference signal from a reference signal storage unit (not illustrated). Then, the FDI-Capon circuit 302 applies the Fourier transform to the reference signal to decompose the reference signal into frequency components ($X_{r1}$, $X_{r2}$, $X_{r3}$, ..., $X_{rN}$).

Then, the FDI-Capon circuit 302 performs whitening represented by the following formula.

$$X_{wk} = \frac{X_{sk} X_{rk}^{*}}{|X_{rk}|^2 + \eta} \quad \cdots\cdots \quad (6)$$

where $X_{wk}$ ($k = 1, 2, ..., N$) indicates frequency components, $\eta$ indicates a minute amount for stabilization of calculation, and * indicates a complex conjugate, after whitening processing. Then, the FDI-Capon circuit 302 calculates a correlation matrix R by using a vector $X_f$ having frequency components that have undergone whitening.

$$X_f = [X_{W1}, X_{W2}, ..., X_{WN}]^T$$

$$R = X_f X_f^{T*}$$

where T indicates transposition. The correlation matrix R is a matrix having a size of N x N. Then, the FDI-Capon circuit 302 extracts partial matrices from the correlation matrix R, and applies the frequency averaging technique to the partial matrices for averaging.

$$R' = \frac{1}{M}\sum_{m=1}^{M} R_m$$

$$R_{mij} = X_{w(i+m-1)}X_{w(j+m-1)}{}^{*} \qquad \ldots\ldots \quad (7)$$

where R' indicates a frequency average correction matrix, $R_m$ indicates a partial matrix of the correlation matrix R, and $R_{mij}$ indicates elements of $R_m$. Thus, the FDI-Capon circuit 302 calculates the frequency average correction matrix R'.

Then, the FDI-Capon circuit 302 inputs the restriction vector C. The restriction vector C varies according to a position r within the processing range, and is defined by the following formula.

$$C = [\exp(jk_1r), \exp(jk_2r), \ldots, \exp(jk_{(N-M+1)}r)]$$

The FDI-Capon circuit 302 calculates a signal strength distribution P (r) in the processing range by using the frequency average correction matrix R' and the restriction vector C. The calculated signal strength distribution P(r) indicates distribution information reflecting the acoustic impedance difference between intra-object tissues (information about distribution related to the acoustic characteristics) according to the present exemplary embodiment.

$$P(r) = \frac{1}{C^{T*}\left(R' + \eta' E\right)^{-1} C}$$

$$\ldots\ldots \quad (8)$$

where $\eta'E$ indicates a diagonal matrix added to stabilize the inverse matrix calculation.

In the present exemplary embodiment, the adaptive signal processing block 007 applies the FDI method and adaptive signal processing (by using the Capon method) to the plurality of receiving signals in this way. As a result, the adaptive signal processing block 007 outputs a signal strength distribution (equivalent to the second distribution information) with an improved resolution in the depth direction.

A third example configuration of the adaptive signal processing block 007 will be described below with reference to Fig. 3C. A delay processing circuit 401 applies the Hilbert transform and the delay processing according to the target positions to the plurality of receiving signals output from the plurality of conversion elements 002, and outputs digital signals. A Capon circuit 402 inputs the digital signals that have undergone the delay processing, and applies the Capon processing to the digital signals. The Capon circuit 402 performs similar processing to the above-described processing (redundant descriptions will be omitted), and eventually outputs a signal Y [s] calculated by the following formula. X' [s] indicates a vector extracted from the input vector X[s] of the s-th sample, fitting the size of the complex weight $W_{opt}$.

$$Y[s] = W_{opt}{}^{H} X'[s] \qquad \ldots\ldots \quad (9)$$

The output Y [s] holds phase information of the reflected waveforms according to the target positions, enabling performing subsequent FDI-Capon processing. The FDI-Capon circuit 302 applies the FDI-Capon processing to the input signal Y[s], and outputs a signal strength distribution.

Performing such processing enables acquiring a power strength distribution with improved resolutions in the depth direction and in the direction perpendicular to the depth direction.

Although the processing of the Capon method has specifically been described as an example of adaptive signal processing, similar effects of the present exemplary embodiment can also be obtained by using other adaptive signal processing, such as the MUSIC method and the ESPRIT method.

<Display method>

Processing performed by a display method according to the present exemplary embodiment will be described below with reference to Fig. 4. Fig. 4 is a flowchart illustrating a display method according to the present exemplary embodiment.

In step S101, the display control unit 008 outputs to the display unit 009 image information for displaying the image of the input first distribution information, and the display unit 009 displays the image of the first distribution information based on the image information.

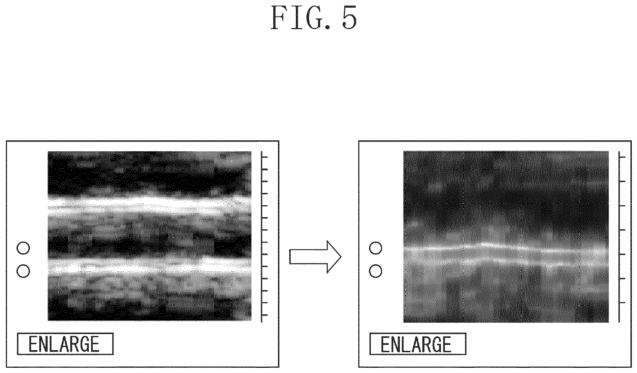

In step S102, the display control unit 008 determines whether an enlargement instruction (enlargement instruction information) is input from the user. The user specifies an area to be enlarged (enlargement area) by using the input unit 010, such as a mouse, while monitoring the image of the first distribution information displayed on the display unit 009. The system control unit 004 inputs enlargement area information from the input unit 010, and outputs the enlargement area information to the display control unit 008 as enlargement instruction information from the user. Thus, to specify an enlargement area, the user inputs an enlargement instruction by specifying a desired area in the image of the first distribution information. Then, the display control unit 008 determines the enlargement rate for the enlarged image based on the relation between the size of the area specified by the user and the size of the display area displayed on the display unit 009. It is also useful to input an enlargement start instruction when the user specifies an enlargement area and then clicks the ENLARGE button (refer to Fig. 5) displayed on the screen of the display unit 009.

However, the enlargement instruction information from the user includes not only the enlargement area information from the user but also the enlargement rate information. The user inputs the enlargement rate by using the input unit 010, such as a dial, while monitoring the image of the first distribution information. The system control unit 004 receives the enlargement rate information from the input unit 010, and outputs the enlargement rate information to the display control unit 008 as enlargement instruction information from the user. When the user inputs the enlargement rate for the image of the first distribution information in this way, the input information serves as an enlargement instruction. The center position for image enlargement may be the center of the image of the first distribution information, or arbitrarily set by the user.

When an enlargement instruction is input (YES in step S102), then in step S103, the display control unit 008 displays the enlarged image. The enlarged image to be displayed is not an enlarged version of the image of the first distribution information but an enlarged version of the image of the second distribution information corresponding to the same position or an enlarged version of the combined image of the first and second distribution information. The present exemplary embodiment will be first described below centering on a case where an enlarged version of the image of the second distribution information is displayed. An example of displaying an enlarged version of the combined image of the first and second distribution information will be described in a third exemplary embodiment (described below).

Fig. 5 illustrates an example screen displayed on the display unit 009 according to the present exemplary embodiment. Fig. 5 illustrate a layer structure of the blood vessel wall. In the example screen, the image on the left is the image before enlargement (image of the first distribution information), and the image on the right is the image after enlargement (image of the second distribution information). In the example illustrated in Fig. 5, the adaptive signal processing block 007 performs processing by combining the FDI method and the Capon method (the example illustrated in Fig. 3B) as adaptive signal processing to acquire the image of the second distribution information.

The scale displayed on the screen illustrated in Fig. 5 is a guide indicating a reduction scale. The screen may display an enlargement rate guide in addition to the reduction scale guide.

The screen also displays the ENLARGE button. When the user clicks the ENLARGE button with an enlargement area specified (with enlargement instruction information input to the display control unit 008), an enlargement start instruction is input to the display control unit 008. In this example, it is assumed that the enlargement instruction information indicates that the user has specified an area above the center of the image of the first distribution information as an enlargement area. Upon reception of the enlargement start instruction, the display control unit 008 outputs to the display unit 009 image information for displaying an enlarged version of the image of the second distribution information. The screen of the display unit 009 changes based on the image information.

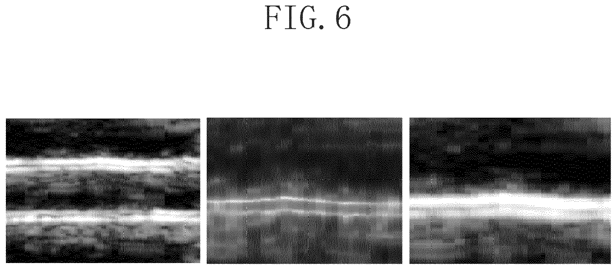

A comparison between an enlarged version of the image of the first distribution information according to the present exemplary embodiment and an enlarged version of the image of the first distribution information to which the present exemplary embodiment is not applied, will be described below. Fig. 6 illustrates an image of the first distribution information before enlargement (left), an enlarged version of the image of the second distribution information generated through the adaptive signal processing according to the present exemplary embodiment (center), and a simply enlarged version of the image of the first distribution information (right) . As illustrated in Fig. 6, depending on the enlargement rate, simply enlarging the image of the first distribution information may not improve image visibility because of a low resolution. However, it turns out that the resolution has been improved for an enlarged version of the image of the second distribution information acquired through the adaptive signal processing.

Although, in the present exemplary embodiment, an enlarged version of the image of the second distribution information is displayed when the user inputs the enlargement instruction information, the effect of the present exemplary embodiment can also be obtained by displaying the combined image of the first and second distribution information. Depending on the enlargement rate, the display control unit 008 may display on the display unit 009 a simply enlarged version of image of the first distribution information. In other words, when the enlargement rate is equal to or larger than a predetermined value, the display control unit 008 may display an enlarged version of the image of the second distribution information or an enlarged version of the combined image of the first and second distribution information. Alternatively, when the enlargement rate is smaller than the predetermined value, the display control unit 008 may display an enlarged version of the image of the first distribution information.

The display control unit 008 may be provided with a function of turning OFF the mode (first mode) for displaying an enlarged version of the image of the second distribution information or an enlarged version of the combined image of the first and second distribution information according to a user instruction regardless of the enlargement rate. In other words, the display control unit 008 may enable selectively executing the mode (first mode) for displaying an enlarged version of the image of the second distribution information or an enlarged version of the combined image of the first and second distribution information, and the mode (second mode) for displaying an enlarged version of the image of the first distribution information according to a user instruction. For example, the user clicks a mode change button displayed on the screen of the display unit 009 to select whether an enlarged image is to be displayed in the first mode or in the second mode. The display control unit 008 receives a mode selection input from the user, and outputs image information of the enlarged image for the selected display mode.

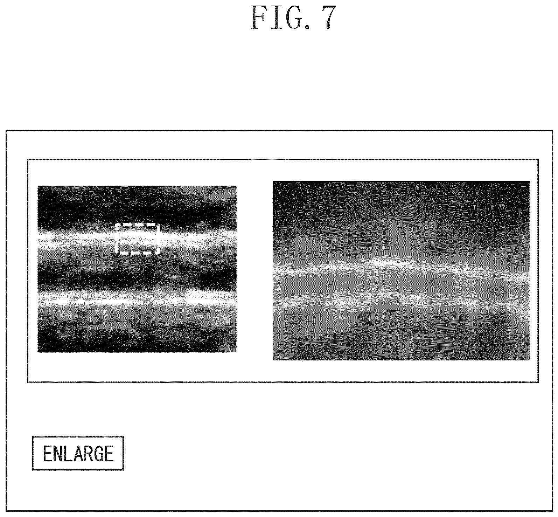

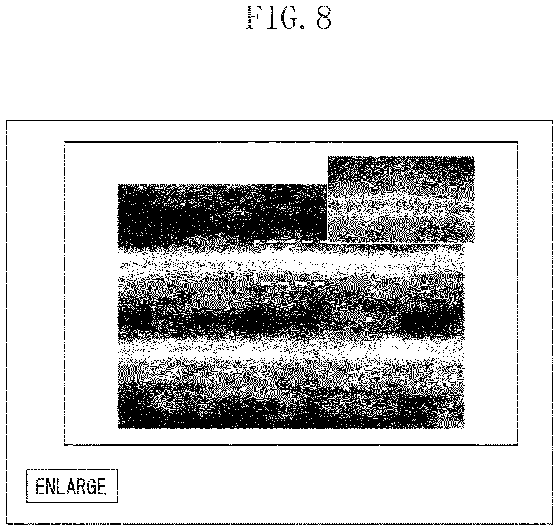

A second exemplary embodiment differs from the first exemplary embodiment in the screen displayed on the display unit 009. An object information acquisition apparatus according to the present exemplary embodiment has a similar configuration to that of the object information acquisition apparatus illustrated in Fig. 1. Since the display method is basically the same as the processing described with reference to Fig. 4, display processing different from that of the first exemplary embodiment will be described with reference to Figs. 7 and 8.

The present exemplary embodiment is characterized in that the first distribution information before enlargement is also displayed on the same screen and that a guide for indicating the position of the enlarged area is displayed, when an enlarged image is displayed. Fig. 7 illustrates an example screen displayed on the display unit 009 according to the present exemplary embodiment. In the present exemplary embodiment, the display control unit 008 also displays a thumbnail of the image of the first distribution information before enlargement in another display area in the screen which displays the enlarged image. A rectangle enclosed by dotted lines indicates the position on the image of the first distribution information corresponding to the enlarged image (the position of the enlargement area on the image of the first distribution information). Displaying a guide for indicating the position of the enlarged area, like this rectangle, makes it easier for the user to grasp the position of the enlarged image.

Instead of displaying the image of the first distribution information before enlargement and the enlarged image in separate display areas, as illustrated in Fig. 7, the display control unit 008 may display the two images in such a manner that at least a part of the image of the first distribution information is overlapped with the enlarged image, as illustrated in Fig. 8. Displaying the enlarged image in the vicinity of the guide for indicating the position of the enlarged area makes it easier for the user to grasp a relation between the two images, improving the operability. This enables reducing the amount of movement of the line of sight between the image before enlargement and the enlarged image, improving the operability.

It is also useful that the position of the enlargement area can be changed when the user moves the guide. When the system control unit 004 receives a guide movement instruction from the user via the input unit 010, the system control unit 004 outputs guide movement information to the display control unit 008. Upon reception of the guide movement information, the display control unit 008 moves the guide on the screen and displays on the display unit 009 an enlarged version of the image in the moved enlargement area.

It is also useful that the size of the enlargement area (i.e., the enlargement rate) can be changed when the user changes the size of the guide. When a guide size change instruction is input from the user to the system control unit 004 via the input unit 010, the system control unit 004 outputs size change information to the display control unit 008. Upon reception of the guide size change information, the display control unit 008 changes the size of the guide on the screen, and displays on the display unit 009 an enlarged version of the image in the changed enlargement area.

Since the position and size of the guide can be changed in this way, the user can easily change the position and size of a target intra-object area to be enlarged, thus improving the operability.

Also in the present exemplary embodiment, similar to the first exemplary embodiment, the display control unit 008 may display on the display unit 009 a simply enlarged version of the image of the first distribution information based on the enlargement rate. More specifically, only when the enlargement rate is larger than a predetermined value, the display control unit 008 displays an enlarged version of the image of the second distribution information or an enlarged version of the combined image of the first and second distribution information. Alternatively, when the enlargement rate is smaller than the predetermined value, the display control unit 008 may display an enlarged version of the image of the first distribution information.

The display control unit 008 may be provided with a function of turning OFF the mode (first mode) for displaying an enlarged version of the image of the second distribution information or an enlarged version of the combined image of the first and second distribution information according to a user instruction regardless of the enlargement rate. In other words, the display control unit 008 may enable selectively executing the mode (first mode) for displaying an enlarged version of the image of the second distribution information or an enlarged version of the combined image of the first and second distribution information, and the mode (second mode) for displaying an enlarged version of the image of the first distribution information according to a user instruction. For example, the user clicks a mode change button displayed on the screen of the display unit 009 to select whether an enlarged image is to be displayed in the first mode or in the second mode. The display control unit 008 receives a mode selection input from the user, and outputs image information of the enlarged image for the selected display mode.

A third exemplary embodiment is characterized in that an enlarged version of the image of the combine image of the first and second distribution information is displayed as an enlarged image. Other processing is similar to that of the first and second exemplary embodiments. An object information acquisition apparatus according to the present exemplary embodiment has a similar configuration to that of the object information acquisition apparatus illustrated in Fig. 1. Since the display method is basically the same as the processing described with reference to Fig. 4, the display processing different from that of the first and second exemplary embodiments will be described.

In the present exemplary embodiment, upon reception of the enlargement instruction information from the user, the display control unit 008 displays in step S103 (Fig. 4) an enlarged version of the combined image of the first and second distribution information. The combination rate of the image of the first distribution information and the image of the second distribution information may be predetermined like 50:50, or arbitrarily set by the user. The combination rate may be changed according to the enlargement rate.

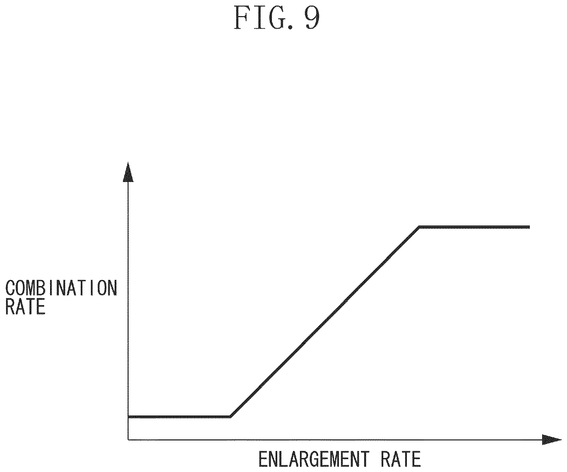

Fig. 9 illustrates an example relation between the enlargement rate and the combination rate. Referring to Fig. 9, when the enlargement rate is below a first predetermined value, the display control unit 008 maintains the combination rate for the first and second distribution information constant. In this case, because of a low enlargement rate, the combination rate of the image of the second distribution information is low (i.e., the ratio of the image of the first distribution information is high, and the ratio of the image of the second distribution information is low in the combined image). Then, when the enlargement rate is higher than the first predetermined value and lower than a second predetermined value, the display control unit 008 increases the combination rate of the image of the second distribution information (the ratio of the image of the second distribution information to the image of the first distribution information in the combined image) with increasing enlargement rate. When the enlargement rate is equal to or higher than the second predetermined value, the display control unit 008 maintains the combination rate for the first and second distribution information constant. In this case, because of a high enlargement rate, the display control unit 008 increases the combination rate for the image of the second distribution information.

Changing the combination rate according to the enlargement rate in this way allows the user to smoothly perform switching between the first and second distribution information without feeling odd, possibly improving the user operability.

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment (s) of the present invention, and by a method performed by the computer of the system or apparatus by, for example, reading out and executing the computer executable instructions from the storage medium to perform the functions of one or more of the above-described embodiment (s) . The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

According to the present invention, using the image obtained by using the adaptive signal processing allows the display unit to display the enlarged image with a high resolution.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

An object information acquisition apparatus according to the present invention includes a fixed signal processing unit configured to perform addition processing with a predetermined weight on a plurality of receiving signals to acquire first distribution information, and an adaptive signal processing unit configured to perform on the plurality of receiving signals adaptive signal processing with a weight adaptively changing according to the receiving signals to acquire second distribution information, wherein the display control unit receives enlargement instruction information for the image of the first distribution information input by the user in a state where the image of the first distribution information is displayed, and outputs image information for displaying on the display unit an enlarged image of the image of the second distribution information or an enlarged image of the combined image of the first and second distribution information.

**Claims**

1.  An object information acquisition apparatus comprising:

a plurality of conversion elements (002) configured to transmit elastic waves to an object, receive reflected waves reflected at respective intra-object positions, and convert the reflected waves into a plurality of receiving signals;

a fixed signal processing means (006) configured to apply addition with a predetermined weight to the plurality of receiving signals to acquire first distribution information;

an adaptive signal processing means (007) configured to apply to the plurality of receiving signals adaptive signal processing with a weight adaptively changing according to the receiving signals to acquire second distribution information; and

a display control means (008) configured to receive the first distribution information and the second distribution information, and output image information for displaying distribution information on a display means (009),

**characterized in that**

the display control means (008) is adapted to receive enlargement instruction information, for the image of the first distribution information, input by the user in a state where the image of the first distribution information is displayed, and output image information for displaying on the display means (009) an enlarged image of the second distribution information or an enlarged image of a combined image of the first and second distribution information.

2. The object information acquisition apparatus according to claim 1, wherein the display control means is configured to enable selectively executing a first mode in which, upon reception of the enlargement instruction information, an enlarged image of the image of the second distribution information or an enlarged version of the combined image is displayed on the display means, and a second mode in which, upon reception of the enlargement instruction information, an enlarged image of the image of the first distribution information is displayed on the display means.

3. The object information acquisition apparatus according to claim 1 or 2, wherein the display control means is configured to display, when the enlargement rate for the first distribution information is equal to or larger than a predetermined value, an enlarged image of the image of the second distribution information or an enlarged image of the combined image and display, when the enlargement rate is smaller than the predetermined value, an enlarged image of the image of the first distribution information.

4. The object information acquisition apparatus according to any one of claims 1 to 3, wherein information about a predetermined area in the image of the first distribution information as the enlargement instruction information is input to the display control means, and the display control means is configured to determine the enlargement rate for the enlarged image based on a relation between a size of an area specified by the user and a size of a display area displayed on the display means, and display an enlarged image of the image in the specified area.

5. The object information acquisition apparatus according to any one of claims 1 to 3, wherein the enlargement instruction information comprises enlargement rate information, and the display control means is configured to display on the display means an enlarged image of the image of the first distribution information enlarged with the enlargement rate centering on a predetermined position in the image of the first distribution information.

6. The object information acquisition apparatus according to any one of claims 1 to 5, wherein the display control means is configured to display the enlarged image in another display area in a same screen as the screen displaying the image of the first distribution information.

7. The object information acquisition apparatus according to any one of claims 1 to 6, wherein the display control means is configured to display, when the enlarged image is displayed, a guide for indicating a position of the enlarged image in the image of the first distribution information.

8. The object information acquisition apparatus according to any one of claims 1 to 7, wherein the adaptive signal processing means is configured to perform processing on the plurality of reception signals so that the electric power is minimized with fixed sensitivity for target directions.

9. The object information acquisition apparatus according to any one of claims 1 to 7, wherein the adaptive signal processing means is configured to perform processing on the plurality of reception signals so that the electric power is minimized with fixed sensitivity for target positions in a depth direction.

10. A display method for displaying an image on a display means (009) by using distribution information acquired by an object information acquisition apparatus, wherein the acquired distribution information includes first distribution information acquired by performing addition processing with a predetermined weight on a plurality of receiving signals acquired by transmitting elastic waves to an object and receiving reflected waves reflected by the object, and second distribution information acquired by performing on the plurality of receiving signals adaptive signal processing with a weight adaptively changing according to the receiving signals, the display method comprising:

> displaying an image of the first distribution information (S101); and
> receiving enlargement instruction information, for the image of the first distribution information, input by a user, and
> **characterized by**

> displaying an enlarged image of the image of the second distribution information or an enlarged image of a combined image of the first and second distribution information (S103).

11. The display method according to claim 10, further comprising selectively executing a first mode in which, upon reception of the enlargement instruction information, an enlarged image of the image of the second distribution information or an enlarged image of the combined image is displayed on the display means; and a second mode in which, upon reception of the enlargement instruction information, an enlarged image of the image of the first distribution information is displayed on the display means.

12. The display method according to claim 10 or 11, wherein, when the enlarged image is displayed, displaying, when an enlargement rate for the first distribution information is equal to or larger than a predetermined value, the enlarged image of the image of the second distribution information or the enlarged image of the combined image; and displaying, when the enlargement rate is smaller than the predetermined value, the enlarged image of the image of the first distribution information.

13. The display method according to any one of claims 10 to 12, further comprising: receiving, after displaying the first distribution information, information about a predetermined area in the image of the first distribution information specified by the user as the enlargement instruction information, and displaying the enlarged image of the image in the predetermined area.

14. The display method according to any one of claims 10 to 12, further comprising: receiving, after displaying the first distribution information, information about the enlargement rate input by the user as the enlargement instruction information, and displaying the enlarged image.

15. The display method according to any one of claims 10 to 14, wherein, when the enlarged image is displayed, displaying the enlarged image in another display area in a same screen as the screen displaying the image of the first distribution information.

16. The display method according to claim 10, wherein, when the enlarged image is displayed, displaying a guide for indicating a position of the enlarged image in the image of the first distribution information.

**Patentansprüche**

1. Objektinformationserhaltevorrichtung mit:

> einer Vielzahl von Umwandlungselementen (002), die konfiguriert sind, elastische Wellen auf ein Objekt zu transmittieren, reflektierte Wellen, die an jeweiligen Positionen innerhalb des Objekts reflektiert werden, zu empfangen, und die reflektierten Wellen in eine Vielzahl von empfangenen Signalen umzuwandeln;
> einer festgelegten Signalverarbeitungseinrichtung (006), die konfiguriert ist, eine Addition mit einer vorbestimmten Wichtung auf die Vielzahl der empfangenen Signale anzuwenden, um eine erste Verteilungsinformation zu erhalten;
> einer adaptiven Signalverarbeitungseinrichtung (007), die konfiguriert ist, ein adaptives Signalverarbeiten mit einer Wichtung, die sich adaptiv gemäß den empfangenen Signalen ändert, auf die Vielzahl der empfangenen Signale anzuwenden, um eine zweite Verteilungsinformation zu erhalten; und
> einer Anzeigesteuerungseinrichtung (008), die konfiguriert ist, die erste Verteilungsinformation und die zweite Verteilungsinformation zu empfangen, und eine Bildinformation zum Anzeigen einer Verteilungsinformation auf einer Anzeigeeinrichtung (009) auszugeben,
**dadurch gekennzeichnet, dass**
die Anzeigesteuerungseinheit (008) angepasst ist, eine Vergrößerungsinstruktionsinformation für das Bild der ersten Verteilungsinformation, die durch den Nutzer in einem Zustand eingegeben wird, indem das Bild der ersten Verteilungsinformation angezeigt wird, zu empfangen, und eine Bildinformation zum Anzeigen eines vergrößerten Bildes der zweiten Verteilungsinformation oder eines vergrößerten Bildes eines kombinierten Bildes aus der ersten und zweiten Verteilungsinformation auf der Anzeigeeinrichtung (009) auszugehen.

2.  Objektinformationserhaltevorrichtung nach Anspruch 1, wobei die Anzeigesteuerungseinrichtung konfiguriert ist, selektiv das Ausführen eines ersten Modus, bei dem beim Empfang der Vergrößerungsinstruktionsinformation ein vergrößertes Bild des Bildes der zweiten Verteilungsinformation oder eine vergrößerte Version des kombinierten Bildes auf der Anzeigeeinrichtung angezeigt wird, und eines zweiten Modus, bei dem beim Empfang der Vergrößerungsinstruktionsinformation ein vergrößertes Bild des Bildes der ersten Verteilungsinformation auf der Anzeigeeinrichtung angezeigt wird, zu ermöglichen.

3.  Objektinformationserhaltevorrichtung nach Anspruch 1 oder 2, wobei die Anzeigesteuerungseinrichtung konfiguriert ist, wenn die Vergrößerungsrate für die erste Verteilungsinformation gleich oder größer als ein vorbestimmter Wert ist, ein vergrößertes Bild des Bildes der zweiten Verteilungsinformation oder ein vergrößertes Bild des kombinierten Bildes anzuzeigen, und, wenn die Vergrößerungsrate kleiner als der vorbestimmte Wert ist, ein vergrößertes Bild des Bildes der ersten Verteilungsinformation anzuzeigen.

4.  Objektinformationserhaltevorrichtung nach einem der Ansprüche 1 bis 3, wobei eine Information über eine vorbestimmte Fläche in dem Bild der ersten Verteilungsinformation als die Vergrößerungsinstruktionsinformation in die Anzeigesteuerungseinrichtung eingegeben wird, und die Anzeigesteuerungseinrichtung konfiguriert ist, die Vergrößerungsrate für das vergrößerte Bild basierend auf einer Beziehung zwischen einer Größe einer durch den Nutzer spezifizierten Fläche und einer Größe einer Anzeigefläche, die auf der Anzeigeeinrichtung angezeigt wird, zu bestimmen, und ein vergrößertes Bild der ersten Bildes in der spezifizierten Fläche anzuzeigen.

5.  Objektinformationserhaltevorrichtung nach einem der Ansprüche 1 bis 3, wobei die Vergrößerungsinstruktionsinformation eine Vergrößerungsrateninformation aufweist, und die Anzeigesteuerungseinrichtung konfiguriert ist, auf der Anzeigeeinrichtung ein vergrößertes Bild des Bildes der ersten Verteilungsinformation, das mit der Vergrößerungsrate vergrößert ist und um eine vorbestimmte Position in dem Bild der ersten Verteilungsinformation zentriert ist, anzuzeigen.

6.  Objektinformationserhaltevorrichtung nach einem der Ansprüche 1 bis 5, wobei die Anzeigesteuerungseinrichtung konfiguriert ist, das vergrößerte Bild in einer anderen Anzeigefläche auf einem gleichen Bildschirm wie dem Bildschirm, der das Bild der ersten Verteilungsinformation anzeigt, anzuzeigen.

7.  Objektinformationserhaltevorrichtung nach einem der Ansprüche 1 bis 6, wobei die Anzeigesteuerungseinrichtung konfiguriert ist, eine Führung zum Anzeigen einer Position des vergrößerten Bildes in dem Bild der ersten Verteilungsinformation anzuzeigen, wenn das vergrößerte Bild angezeigt wird.

8.  Objektinformationserhaltevorrichtung nach einem der Ansprüche 1 bis 7, wobei die adaptive Signalverarbeitungseinrichtung konfiguriert ist, ein Verarbeiten der Empfangssignale so durchzuführen, dass die elektrische Leistung mit einer festgelegten Sensitivität für Zielrichtungen minimiert wird.

9.  Objektinformationserhaltevorrichtung nach einem der Ansprüche 1 bis 7, wobei die adaptive Signalverarbeitungseinrichtung konfiguriert ist, ein Verarbeiten der Vielzahl der Empfangssignale so durchzuführen, dass die elektrische Leistung mit einer festgelegten Sensitivität für Zielpositionen in einer Tiefenrichtung minimiert wird.

10. Anzeigeverfahren zum Anzeigen eines Bildes auf einer Anzeigeeinrichtung (009) unter Verwendung einer Verteilungsinformation, die durch eine Objektinformationerhaltevorrichtung erhalten wird, wobei die erhaltene Verteilungsinformation eine erste Verteilungsinformation, die durch Durchführen einer Additionsverarbeitung mit einer vorbestimmten Wichtung auf einer Vielzahl von empfangenen Signalen, die durch Transmittieren von elastischen Wellen auf ein Objekt und Empfangen von reflektierten Wellen, die durch das Objekt reflektiert werden, erhalten werden und eine zweite Verteilungsinformation, die durch Durchführen eines adaptive Signalverarbeitens mit einer sich adaptiv ändernden Wichtung gemäß den empfangenen Signalen erhalten wird, aufweist, wobei das Anzeigeverfahren aufweist:

Anzeigen eines Bildes der ersten Verteilungsinformation (S101); und

Empfangen einer Vergrößerungsinstruktionsinformation für das Bild der ersten Verteilungsinformation, die durch einen Nutzer eingegeben wird, und

**gekennzeichnet durch**

Anzeigen eines vergrößerten Bildes des Bildes der zweiten Verteilungsinformation oder eines vergrößerten Bildes eines kombinierten Bildes der ersten und zweiten Verteilungsinformation (S103).

11. Anzeigeverfahren nach Anspruch 10, ferner mit einem selektiven Ausführen eines ersten Modus, in dem beim Empfang der Vergrößerungsinstruktionsinformation ein vergrößertes Bild des Bildes der zweiten Verteilungsinformation oder ein vergrößertes Bild des kombinierten Bildes auf der Anzeige Einrichtungen angezeigt wird, und eines zweiten Modus, bei dem beim Empfang der Vergrößerungsinstruktionsinformation ein vergrößertes Bild des Bildes der ersten Verteilungsinformation auf der Anzeigeeinrichtung angezeigt wird.

12. Anzeigeverfahren nach Anspruch 10 oder 11, wobei, wenn das vergrößerte Bild angezeigt wird:

wenn eine Vergrößerungsrate für die erste Verteilungsinformation gleich oder größer als ein vorbestimmter Wert ist, das vergrößerte Bild des Bildes der zweiten Verteilungsinformation oder das vergrößerte Bild des kombinierten Bildes angezeigt wird; und

wenn die Vergrößerungsrate kleiner als der vorbestimmte Wert ist, das vergrößerte Bild des Bildes der ersten Verteilungsinformation angezeigt wird.

13. Anzeigeverfahren nach einem der Ansprüche 10 bis 12, ferner mit:

Empfangen einer Information über eine vorbestimmte Fläche in dem Bild der ersten Verteilungsinformation, die durch den Nutzer spezifiziert wird, als die vergrößerte Instruktionsinformation, nach einem Anzeigen der ersten Verteilungsinformation, und Anzeigen des vergrößerten Bildes des Bildes in der vorbestimmten Fläche.

14. Anzeigeverfahren nach einem der Ansprüche 10 bis 12, ferner mit:

Empfangen einer Information über die Vergrößerungsrate, die durch den Nutzer eingegeben wird, als die Vergrößerungsinstruktionsinformation nach einem Anzeigen der ersten Verteilungsinformation, und Anzeigen des vergrößerten Bildes.

15. Anzeigeverfahren nach einem der Ansprüche 10 bis 14, wobei, wenn das vergrößerte Bild angezeigt wird, das vergrößerte Bild in einer anderen Anzeigefläche in einem gleichen Bildschirm wie dem Bildschirm, der das Bild der ersten Verteilungsinformation anzeigt, angezeigt wird.

16. Anzeigeverfahren nach Anspruch 10, wobei, wenn das vergrößerte Bild angezeigt wird, eine Führung zum Angeben einer Position des vergrößerten Bildes in dem Bild der ersten Verteilungsinformation angezeigt wird.

**Revendications**

1. Appareil d'acquisition d'informations d'objet, comprenant :

une pluralité d'éléments de conversion (002) configurés pour émettre des ondes élastiques vers un sujet, recevoir des ondes réfléchies, réfléchies à des positions internes à l'objet respectives, et convertir les ondes réfléchies en une pluralité de signaux de réception ;

un moyen de traitement fixe de signaux (006) configuré pour appliquer une addition avec un poids prédéterminé à la pluralité de signaux de réception de façon à acquérir des premières informations de distribution ;

un moyen de traitement adaptatif de signaux (007) configuré pour appliquer, à la pluralité de signaux de réception, un traitement adaptatif de signaux avec un poids variant de manière adaptative conformément aux signaux de réception de façon à acquérir des secondes informations de distribution ; et

un moyen de commande d'affichage (008) configuré pour recevoir les premières informations de distribution et les secondes informations de distribution, et pour délivrer en sortie des informations d'image à des fins d'affichage d'informations de distribution sur un moyen d'affichage (009),

**caractérisé en ce que**

le moyen de commande d'affichage (008) est apte à recevoir des informations d'instruction d'agrandissement, concernant l'image des premières informations de distribution, entrées par l'utilisateur dans un état dans lequel est affichée l'image des premières informations de distribution, et à délivrer en sortie des informations d'image à des fins d'affichage sur le moyen d'affichage (009) d'une image agrandie des secondes informations de distribution ou d'une image agrandie d'une image combinée des premières et secondes informations de distribution.

2. Appareil d'acquisition d'informations d'objet selon la revendication 1, dans lequel le moyen de commande d'affichage est configuré pour permettre une exécution sélective d'un premier mode dans lequel, lors de la réception des informations d'instruction d'agrandissement, une image agrandie de l'image des secondes informations de distribution ou une version agrandie de l'image combinée est affichée sur le moyen d'affichage, et d'un second mode dans lequel, lors de la réception des informations d'instruction d'agrandissement, une image agrandie de l'image des premières informations de distribution est affichée sur le moyen d'affichage.

3. Appareil d'acquisition d'informations d'objet selon la revendication 1 ou 2, dans lequel le moyen de commande d'affichage est configuré pour afficher, lorsque le taux d'agrandissement des premières informations de distribution est égal ou supérieur à une valeur prédéterminée, une image agrandie de l'image des secondes informations de distribution ou une image agrandie de l'image combinée et pour afficher, lorsque le taux d'agrandissement est inférieur à la valeur prédéterminée, une image agrandie de l'image des premières informations de distribution.

4. Appareil d'acquisition d'informations d'objet selon l'une quelconque des revendications 1 à 3, dans lequel des informations concernant une zone prédéterminée de l'image des premières informations de distribution, en tant qu'informations d'instruction d'agrandissement, sont entrées dans le moyen de commande d'affichage, et le moyen de commande d'affichage est configuré pour déterminer le taux d'agrandissement de l'image agrandie sur la base d'une relation entre une taille d'une zone spécifiée par l'utilisateur et une taille d'une zone d'affichage affichée sur le moyen d'affichage, et pour afficher une image agrandie de l'image dans la zone spécifiée.

5. Appareil d'acquisition d'informations d'objet selon l'une quelconque des revendications 1 à 3, dans lequel les informations d'instruction d'agrandissement comprennent des informations de taux d'agrandissement, et le moyen de commande d'affichage est configuré pour afficher, sur le moyen d'affichage, une image agrandie de l'image des premières informations de distribution, agrandie conformément au taux d'agrandissement, centrée sur une position prédéterminée de l'image des premières informations de distribution.

6. Appareil d'acquisition d'informations d'objet selon l'une quelconque des revendications 1 à 5, dans lequel le moyen de commande d'affichage est configuré pour afficher l'image agrandie dans une autre zone d'affichage d'un même écran que l'écran affichant l'image des premières informations de distribution.

7. Appareil d'acquisition d'informations d'objet selon l'une quelconque des revendications 1 à 6, dans lequel le moyen de commande d'affichage est configuré pour afficher, lorsque l'image agrandie est affichée, un guide destiné à indiquer une position de l'image agrandie dans l'image des premières informations de distribution.

8. Appareil d'acquisition d'informations d'objet selon l'une quelconque des revendications 1 à 7, dans lequel le moyen de traitement adaptatif de signaux est configuré pour appliquer un traitement à la pluralité de signaux de réception de façon à réduire à un minimum la puissance électrique avec une sensibilité fixée pour des directions cibles.

9. Appareil d'acquisition d'informations d'objet selon l'une quelconque des revendications 1 à 7, dans lequel le moyen de traitement adaptatif de signaux est configuré pour appliquer un traitement à la pluralité de signaux de réception de façon à réduire à un minimum la puissance électrique avec une sensibilité fixée pour des positions cibles dans une direction de profondeur.

10. Procédé d'affichage permettant d'afficher une image sur un moyen d'affichage (009) au moyen d'informations de distribution acquises par un appareil d'acquisition d'informations d'objet, dans lequel les informations de distribution acquises comprennent des premières informations de distribution acquises par une application d'un traitement d'addition avec un poids prédéterminé à une pluralité de signaux de réception acquis par une émission d'ondes élastiques vers un objet et une réception d'ondes réfléchies, réfléchies par l'objet, et des secondes informations de distribution acquises par une application, à la pluralité de signaux de réception, d'un traitement adaptatif de signaux avec un poids variant de manière adaptative conformément aux signaux de réception, le procédé d'affichage comprenant les étapes consistant à :

afficher une image des premières informations de distribution (S101) ; et
recevoir des informations d'instruction d'agrandissement, concernant l'image des premières informations de distribution, entrées par un utilisateur, et

**caractérisé par** l'étape consistant à

afficher une image agrandie de l'image des secondes informations de distribution ou une image agrandie d'une image combinée des premières et secondes informations de distribution (S103).

11. Procédé d'affichage selon la revendication 10, comprenant en outre l'étape consistant à exécuter sélectivement un premier mode dans lequel, lors d'une réception des informations d'instruction d'agrandissement, une image agrandie de l'image des secondes informations de distribution ou une image agrandie de l'image combinée est affichée sur le moyen d'affichage ; et un second mode dans lequel, lors d'une réception des informations d'instruction d'agrandissement, une image agrandie de l'image des premières informations de distribution est affichée sur le moyen d'affichage.

12. Procédé d'affichage selon la revendication 10 ou 11, où, lorsque l'image agrandie est affichée, le procédé comprend les étapes consistant à :

afficher, lorsqu'un taux d'agrandissement des premières informations de distribution est égal ou supérieur à une valeur prédéterminée, l'image agrandie de l'image des secondes informations de distribution ou l'image agrandie de l'image combinée ; et

afficher, lorsque le taux d'agrandissement est inférieur à la valeur prédéterminée, l'image agrandie de l'image des premières informations de distribution.

13. Procédé d'affichage selon l'une quelconque des revendications 10 à 12, comprenant en outre les étapes consistant à : recevoir, après l'affichage des premières informations de distribution, des informations concernant une zone prédéterminée de l'image des premières informations de distribution spécifiées par l'utilisateur en tant qu'informations d'instruction d'agrandissement, et afficher l'image agrandie de l'image dans la zone prédéterminée.

14. Procédé d'affichage selon l'une quelconque des revendications 10 à 12, comprenant en outre les étapes consistant à : recevoir, après l'affichage des premières informations de distribution, des informations concernant le taux d'agrandissement entrées par l'utilisateur en tant qu'informations d'instruction d'agrandissement, et afficher l'image agrandie.

15. Procédé d'affichage selon l'une quelconque des revendications 10 à 14, où, lorsque l'image agrandie est affichée, le procédé comprend l'étape consistant à afficher l'image agrandie dans une autre zone d'affichage d'un même écran que l'écran d'affichage de l'image des premières informations de distribution.

16. Procédé d'affichage selon la revendication 10, où, lorsque l'image agrandie est affichée, le procédé comprend l'étape consistant à

afficher un guide destiné à indiquer une position de l'image agrandie dans l'image des premières informations de distribution.

FIG. 1

FIG. 2

FIG. 3A

FIG. 3B

FIG. 3C

# FIG. 4

START

DISPLAY IMAGE OF FIRST
DISTRIBUTION INFORMATION — S101

IS
INFORMATION ABOUT
ENLARGEMENT INSTRUCTION
INPUT? — S102

NO

YES

DISPLAY ENLARGED IMAGE — S103

END

20

FIG. 5

FIG. 6

FIG. 7

ENLARGE

# FIG. 8

ENLARGE

# FIG. 9

COMBINATION
RATE

ENLARGEMENT RATE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012024133 A
- US 5471989 A
- US 20120022373 A1
- US 20110307187 A1
- JP H07397 A

### Non-patent literature cited in the description

- **M. SASSO et al.** Medical Ultrasound Imaging Using The Fully Adaptive Beamformer. *Proc. Acoustics, Speech Signal Process,* March 2005, vol. 2, 489-492
- **HIROFUMI TAKI ; KOUSUKE TAKI ; TAKUYA SAKAMOTO ; MAKOTO YAMAKAWA ; TSUYOSHI SHIINA ; TORU SATO.** *Conf Proc IEEE Eng Med Biol Soc.,* 2010, vol. 1, 5298-5301